# DEMANDE DE BREVET EUROPEEN

(11) **EP 0 865 786 A1**
(43) Date de publication de la demande: **23.09.1998**
(21) Numéro de dépôt: 98400399.6
(22) Date de dépôt: 19.02.1998
(51) Int. Cl.: A61K 7/48, A61K 7/06, A61K 7/32, A61K 7/02

(54) **Composition aqueuse topique solide ayant l'aspect d'un gel permettant la formation d'un film lors de son application**

(30) Priorité: 14.03.1997 FR 9703119
(71) Demandeur: L'OREAL, 75008 Paris (FR)
(72) Inventeur: Dupuis, Christine, 75018 Paris (FR); Samain, Henri, 91570 Bievres (FR); Roulier, Véronique, 75010 Paris (FR); Ferrari, Véronique, 94700 Maisons Alfort (FR)
(74) Mandataire: Miszputen, Laurent

(57) **Abrégé**

La présente invention concerne une composition aqueuse solide, en particulier une composition topique solide, constituée par une matrice ayant l'aspect d'un gel présentant le profil rhéologique suivant:
- une viscosité initiale au repos V₀ suffisante pour former une composition solide, de préférence comprise entre 50 000 et 1 000 000 Pa.s, la dite viscosité V₀ étant stable jusqu'à une contrainte de cisaillement C₁,
- une viscosité V2 après cisaillement à une contrainte C₂ pour laquelle le rapport V₀/V₂ est supérieur ou égal à 1 000,
- la différence C₂-C₁ étant inférieure ou égale à 1 000 Pa.
La composition selon l'invention peut être utile comme composition de maquillage, de coiffage, d'hygiène corporelle ou de soin.

## Description

La présente invention concerne une nouvelle composition aqueuse solide, en particulier topique, constituée par une matrice ayant l'aspect d'un gel aqueux, permettant la formation d'un film lors de son application.

On connaît dans l'industrie cosmétique divers types de produits sous forme d'un solide notamment dans le domaine du maquillage comme les bâtons ou "sticks" de rouge à lèvres, de fond de teint ou d'ombre à paupières, dans le domaine du soin de la peau ou des lèvres comme les crayons réparateurs des lèvres, les bâtons ou "sticks" dépigmentants ou hydratants, et dans le domaine de l'hygiène comme les sticks déodorants. L'application de la composition se fait par abrasion de la composition solide, ce qui rend difficile la formation de films continus durables.

Ce problème de la formation de films continus par l'application d'une composition solide est particulièrement important pour les compositions de maquillage qui sont composées d'un mélange hétérogène de cires, d'huiles et de poudres (charges et pigments), plus particulièrement pour les compositons de maquillage dites « sans transfert », c'est à dire dont les constituants ne transfèrent pas sur des supports avec lesquels ils peuvent entrer en contact (par exemple des tissus, des verres, des tasses, etc). d'autres . En effet, la réalisation d'une composition de maquillage « sans transi fert » nécessite l'emploi d'une composition complexe dans laquelle les huiles sont partiellement remplacées par des solvants volatils qui s'évaporent au contact de la peau pour laisser une couche composée essentiellement de cires et/ou de résines et de pigments et charges (JP 62-61911, JP 61-65 809, EP 602 905). Cette solution, outre les difficultés de préparation liées à l'emploi de composés volatils, présente l'inconvénient de conduire à un maquillage d'aspect poudreux et mat.

La Demanderesse a maintenant trouvé qu'en employant une matrice solide ayant l'aspect d'un gel aqueux présentant un profil rhéologique particulier, il était possible d'obtenir une composition aqueuse solide, permettant la formation d'un film après son application et séchage, apportant une solution aux problèmes exposés ci-dessus.

La présente invention concerne donc une composition topique solide aqueuse constituée par une matrice solide ayant l'aspect d'un gel aqueux présentant le profil rhéologique suivant:
- une viscosité initiale au repos V₀ suffisante pour former une composition solide, de préférence comprise entre 50 000 et 1 000 000 Pa.s, la dite viscosité V₀ étant stable jusqu'à une contrainte de cisaillement C₁,
- une viscosité V₂ après cisaillement à une contrainte C₂ pour laquelle le rapport V₀/V₂ est supérieur ou égal à 1 000,
- la différence C₂ -C₁ étant inférieure ou égale à 1 000 Pa.

La matrice solide a une viscosité initiale stable, c'est à dire constante sous de faibles contraintes de cisaillement de manière que la composition puisse être manipulée sans entraîner de modification importante de sa viscosité. Cette stabilité de la viscosité initiale est exprimée par une valeur de la viscosité du gel V₁ mesurée à la containte de cisaillement C₁ proche de V₀. Il est entendu que cette proximité doit être appréciée au regard de la chute de viscosité après cisaillement. De manière avantageuse, le rapport V₀/V₁ est inférieur ou égal à 2.

La contrainte de cisaillement C₁ est caractéristique de la force nécessaire pour obtenir une fluidification de la matrice solide, permettant son étalement sur le support sur lequel on l'applique, peau, cheveux, lèvres, etc. De préférence, la contrainte de cisaillement C₁ est supérieure ou égale à 500 Pa. Toutefois, l'homme du métier saura déterminer la valeur que ne devra pas dépasser cette contrainte de cisaillement C₁ pour ne pas altérer ce support.

Ainsi, pour une application sur les cheveux, la contrainte de début de cisaillement C₁ est de de manière avantageuse inférieure ou égale à 1 500 Pa.

Il est entendu ci-dessus et ci-après que les différentes valeurs de viscosité et de contrainte sont mesurées une fois la matrice formée. Avant d'effectuer les mesures de viscosité et de profil rhéologique de la composition, il convient de s'assurer que la matrice soit bien formée et stable. Il convient donc d'attendre au moins 24 heures après la préparation de la matrice.

De manière préférentielle, les chutes de viscosité induites par le cisaillement sur la composition ne sont pas immédiatement réversibles, c'est à dire que la matrice ne se casse pas sous forte contrainte et reste homogène lorsqu'elle a atteint sa viscosité la plus faible.

De manière avantageuse, cette matrice est formée par l'addition d'un matériau gélifiant hydrophile approprié.

Le matériau gélifiant hydrophile selon l'invention est constitué par tout matériau gélifiant susceptible de former une matrice solide ayant l'apparence d'un gel et présentant le profil rhéologique selon l'invention.

Selon un mode de réalisation préférentiel de l'invention, le matériau gélifiant hydrophile est un polymère gélifiant hydrophile. Des polymères gélifiants hydrophiles utiles selon l'invention sont en particulier des polyesters sulfonés de masse moléculaire en poids inférieure à 20 000, de préférence inférieure à 15 000.

De tels polymères peuvent être plus particulièrement des oligomères copolyesters terephtaliques hydrosolubles ou hydrodispersables comprenant essentiellement des motifs répétitifs dicarboxylates de formule (I):

[-CO-A-CO-O-(CH₂-CH₂)ₙ-] (I)

dans laquelle
A représente un groupement 1,4-phénylène, sulfo-1,3-phénylène et éventuellement 1,3-phénylène,
n va de 1 à 4,
au moins 35 % en mole desdits motifs de formule (I) étant des motifs de formule (I) pour lesquels A représente un groupement 1,4-phénylène et n est égal à 1,
au moins 7 % en mole desdits motifs de formule (I) étant des motifs de formule (I) pour lesquels A représente un groupement sulfo-1,3-phénylène,
la masse moléculaire en poids desdits oligomères copolyesters étant inférieure à 20 000, de préférence inférieure à 15 000.

De manière préférentielle, au moins 40 % en mole, plus préférentiellement entre 40 et 90 % en mole des motifs de formule (I) sont des motifs de formule (I) pour lesquels A représente un groupement 1,4-phénylène et n est égal à 1.

De préférence, au moins 10 % en mole, plus préférentiellement entre 10 % et 25 % en mole des motifs de formule (I) sont des motifs de formule (I) pour lesquels A représente un groupement sulfo-1,3-phénylène.

Les extrémités des chaînes desdits oligomères copolyesters peuvent être semblables ou différentes et représentées essentiellement par les groupements de formule (I'):

-CO-A-CO-O-(CH₂-CH₂)ₙ-OH (I')

dans laquelle A et n sont définis ci-dessus.

Lesdits oligomères peuvent également présenter en extrêmités de chaîne, et ce en quantités mineures des groupements de formules

-A-CO-OH

-A-CO-OR

formules dans lesquelles A est défini ci-dessus et R représente un groupement alkyle en C₁-C₄.

Lorsque A représente un groupement sulfo-1,3-phénylène, il s'agit plus particulièrement d'un sulfonate de métaux alcalins, notamment sodium ou potassium, ou d'un sulfonate d'ammonium ou de mono-, di-, tri- ou tétra- alkylammonium inférieur. Par alkylammonium inférieur, on entend de préférence selon l'invention un ammonium dont le ou les radicaux alkyles sont des alkyles inférieurs, de préférence en C₁-C₆. De manière préférentielle, il s'agit d'un sulfonate de sodium.

L'oligomère copolyester peut comprendre éventuellement jusqu'à 20 % en mole, de préférence jusqu'à 5 % en mole de motifs de formule (I) pour lesquels A représente un groupement 1,3-phénylène.

Selon un mode de réalisation préférentiel de l'invention, l'oligomère copolyester ci-dessus a une masse moléculaire en poids comprise entre 5 000 et 14 000, plus préférentiellement comprise entre 8 000 et 10 000.

Les masses moléculaires en poids sont mesurées par chromatographie par perméation de gel dans le diméthylacétamide contenant 10⁻² N de LiBr, à 100°C. Les résultats sont exprimés en équivalents polystyrène.

Lesdits oligomères copolyesters peuvent être obtenus par les procédés usuels de préparation des polyesters par voie fondue, voie solvant ou voie interfaciale, procédés faisant intervenir des réactions
. d'estérification de diacides et de diols et polycondensation
. de transestérification de diesters et de diols et polycondensation
. d'autocondensation d'hydroxyacides
. de Schotten-Baumann par mise en oeuvre de diols et de chlorures d'acide et polycondensation
. de polymérisation de lactones en contrôlant la teneur minimum en motifs de formule (I) pour lesquels A représente un groupement 1,4-phénylène et n est égal à 1, semblables par les rapports stoechiométriques initiaux des différents monomères et par la maîtrise des réactions secondaires.

Un mode de préparation particulièrement intéressant est celui par transestérification / polycondensation et / ou d'estérification / polycondensation par voie fondue à l'aide d'un catalyseur de transestérification et/ou estérification.

La maîtrise de la structure est obtenue par contrôle de la teneur minimum en motifs de formule (I) pour lesquels A représente un groupement 1,4-phénylène et n est égal à 1, semblables par les rapports stoechiométriques initiaux des différents monomères diacides et/ou diesters et diol et par mise en oeuvre d'un agent limiteur d'éthérification, agent limiteur qui peut être un composé basique tel que les amines aliphatiques ou aromatiques ou un hydroxyde ou acétate de métaux alcalins ou alcalino-terreux.

Le contrôle de la masse moléculaire est obtenu d'une manière connue de l'homme du métier, par compromis adéquat entre la pression, la température et le temps.

Les nouveaux oligomères copolyesters téréphtaliques faisant l'objet de l'invention peuvent être préparés par estérification et/ou transestérification / polycondensation d'une composition monomère à base:
- d'acide, anhydride ou diester téréphtalique (Tp)
- d'acide, anhydride ou diester sulfoisophtalique (Slp)
- éventuellement d'acide, anhydride ou diester isophtalique (Ip)
- et d'éthylène glycol (EG)
selon des quantités relatives correspondant à
* un rapport molaire (Slp) / [(Tp)+(SIp)+(Ip)] d'au moins 7/100, de préférence d'au moins 10/100, tout particulièrement de 10/100 à 25/100
* un rapport molaire (Ip) / [(Tp)+(SIp)+(Ip)] de 20/100 au plus, de préférence de 5/100 au plus
* un rapport molaire (EG) / [(Tp)+(SIp)+(Ip)] de 2/1 à 3/1. en présence d'un catalyseur d'estérification et/ou transestérification et d'un limiteur de d'éthérification.

Le monomère téréphtalique (Tp) est de préférence mis en oeuvre sous la forme de diester inférieur (diester de dialkyle en C₁-C₄), de diméthyle de préférence.

Le monomère sulfoisophtalique (Slp) est de préférence mis en oeuvre sous la forme d'un sulfonate de métal alcalin (sodium notamment) de diester inférieur (d'alkyle en C₁-C₄), de méthyle de préférence. On peut citer tout particulièrement le sodio-oxysulfonyl-5 isophtalate de diméthyle.

Le monomère isophtalique (Ip) éventuel est de préférence mis en oeuvre sous la forme d'acide isophtalique.

Lorsque tous les monomères "diacides" sont mis en oeuvre sous la forme d'un diesters, l'opération de transestérification (interéchange) entre ces monomères "diacides" et l'éthylène glycol est effectuée à une température supérieure ou égale à 130°C, de préférence de l'ordre 140 à 220°C et tout particulièrement de l'ordre de 180 à 220°C ; à cette température le méthanol (cas préférentiel des diesters méthyliques) formé est éliminé du milieu réactionnel de préférence par distillation.

Cette opération d'interéchange est réalisée en présence d'un catalyseur de transestérification métallique et d'un limiteur d'éthérification. Ledit catalyseur est de préférence un carboxylate métallique, tel que l'acétate de manganèse, l'acétate de zinc, l'acétate de cobalt ou l'acétate de calcium, ou d'un titanate organique ou minéral, tel que le titanate de butyle, le titanate de nitrilo-2,2',2"-triéthyle (ou aminotriéthanolate de titane jouant en outre le rôle de limiteur d'éthérification) ou le titanate de calcium. Les catalyseurs préférés sont les titanates organiques ; ils sont mis en oeuvre en quantités de l'ordre d'au moins 0,001% en poids exprimé en titane, de préférence de l'ordre de 0,002% à 0,02% en poids de titane par rapport au poids de réactifs présents.

L'agent limiteur d'éthérification peut être un composé basique tel que les amines aliphatiques ou aromatiques (triéthanolamine, carbonate de guanidine, diméthylaniline, naphtylamine ...) ou un hydroxyde ou acétate de métaux alcalins ou alcalino-terreux (acétate de sodium, potassium, benzoate de sodium ...). Il est mis en oeuvre généralement en quantité de l'ordre de 0,001% à 0,05% par rapport au poids de réactifs présents.

La durée de l'opération d'interéchange est de 1 à 4 heures ; elle est généralement de l'ordre de 2 à 3 heures.

Lorsque plus de 90% de la quantité théorique de méthanol a été distillée, le polyol excédentaire est éliminé en portant la température du milieu réactionnel à 230°C.

L'opération de polycondensation est de préférence réalisée à une température de l'ordre de 230 à 280°C, de préférence de l'ordre de 240 à 260°C, dans un autre réacteur préalablement porté à cette température et progressivement mis sous vide jusqu'à une pression qui peut aller jusqu'à 10 Pa ; une réduction de pression jusqu'à 10 millibar environ dure de l'ordre de 40 minutes.

L'opération de polycondensation se déroule avec élimination de molécules de polyol, cette opération est stoppée lorsque le couple moteur de l'arbre d'agitation indique une valeur équivalente à environ 0,5 à 5 mètres.newton pour une température de 250°C de la masse réactionnelle et une vitesse d'agitation de 80 tours / minute d'un mobile en forme d'ancre dans un réacteur de 7,5 litres. Le vide est ensuite cassé à l'azote, et le polymère est coulé dans une lingotière ; après refroidissement, le polymère est broyé.

Lorsque l'un des monomères "diacides" est présent sous forme diacide ou anhydride et le ou les autres sous forme de diester(s), lesdits oligomères copolyesters sont obtenus en réalisant d'abord une opération de transestérification des monomères diesters avec de l'éthylène glycol dans les conditions ci-dessus décrites, suivie d'une opération d'estérification dans le milieu du monomère diacide ou anhydride avec de l'éthylène glycol, puis polycondensation dans les conditions décrites ci-dessus, la quantité totale d'éthylène glycol étant répartie entre les deux opérations (transestérification et estérification).

Si nécessaire, l'opération d'estérification est réalisée par ajout dans le milieu réactionnel résultant de l'opération de transestérification, du monomère sous forme diacide ou anhydride et d'éthylène glycol préalablement mis en suspension, à une température correspondant à celle de la fin de la température d'interéchange; la période d'introduction est de l'ordre de 1 heure.

Cette opération d'estérification est réalisée à une température de l'ordre de 230 à 280°C, de préférence de l'ordre de 250 à 260°C, en présence d'un catalyseur du même type que celui de transestérification et d'un agent limiteur d'éthérification.

L'opération est réalisée en présence des mêmes types de catalyseur et de limiteur d'éthérification que ceux mis en oeuvre lors de l'opération de transestérifiacation, et ce dans les mêmes proportions.

La réaction s'effectue avec élimination d'eau qui est soutirée du réacteur en même temps que le polyol en excès.

Ce type de procédé de préparation est notamment décrit dans la demande de brevet WO 95/32997 (RHONE-POULENC CHIMIE).

De manière préférentielle, la composition selon l'invention comprend entre 10 et 40 % en poids par rapport au poids total de la composition de matériau gélifiant hydrophile, plus préférentiellement entre 10 et 30 % en poids.

La composition topique selon l'invention est de manière préférentielle une composition cosmétique ou pharmaceutique, destinée à être appliquée sur la peau, les muqueuses ou les phanères (cheveux, cils, ongles, etc).

Elle peut être employée pour toutes les utilisations dermo-cosmétiques usuelles, et notamment comme composition d'hygiène corporelle, par exemple sous forme de sticks déodorants, comme composition capillaire, par exemple comme stick de coiffage, comme composition de maquillage telle que rouge à lèvres, fond de teint, fard à paupières, etc..., en particulier de maquillage dit « sans transfert », ou encore comme composition de soin.

La composition selon l'invention peut donc comprendre d'autres constituants usuels en cosmétique selon l'utilisation qui est envisagée. Bien entendu, la composition selon l'invention ne comprendra pas de constituants en quantités susceptibles d'altérer les propriétés rhéologiques spécifiques de la matrice solide la constituant.

La composition selon l'invention peut comprendre en outre une phase grasse. La phase grasse peut comprendre des huiles ou cires usuelles en cosmétique, d'origine animale, végétale, minérale ou synthétique, seules ou en mélanges. Dans ce cas, la phase grasse peut être dispersée dans le gel, en particulier sous la forme d'une émulsion de type huile dans eau ou eau dans huile.

La composition selon l'invention peut comprendre des additifs et/ou des actifs usuels en dermo-cosmétique, étant entendu que l'homme du métier saura déterminer les quantités de ces additifs et actifs susceptibles d'être ajoutés à la composition selon l'invention de manière à ne pas altérer le profil rhéologique de la matrice solide la constituant.

Les additifs usuels en cosmétique sont en particulier des parfums, des colorants, des absorbeurs d'odeur, des additifs assurant la stabilité de la composition comme des conservateurs, des filtres U.V.A et/ou U.V.B., des antioxydants hydrophiles et/ou lipophiles, des chélatants, etc. Les quantités de ces différents adjuvants sont celles classiquement utilisées dans le domaine considéré, et par exemple de 0,0001 à 5 % en poids du poids total de la composition. Ces adjuvants, selon leur nature, peuvent être introduits dans la phase aqueuse ou dans la phase grasse lorsque la composition comprend également une phase grasse.

La composition selon l'invention peut également comprendre des actifs usuels en cosmétique, hydrophiles et/ou lipophiles, en particulier des agents anti-radicaux libres, des alpha- ou bêta-hydroxyacides, des filtres U.V.A et/ou U.V.B., des céramides, des agents antipelliculaires comme l'octopirox ou la pyrithione de zinc, des agents antiacnéiques comme l'acide rétinoïque ou le peroxyde de benzoyle, des agents anti-chute des cheveux comme le minoxidil, des agents antifongiques ou antiseptiques, etc.

Pour une utilisation comme composition d'hygiène corporelle, la composition selon l'invention peut comprendre des produits déodorants contenant des substances actives de type antitranspirant et/ou de type bactéricide pour diminuer voire supprimer les odeurs axillaires généralement désagréables, et/ou des absorbeurs d'odeurs.

Les substances antitranspirantes ont pour effet de limiter le flux sudoral. Elles sont notamment constituées de sels d'aluminium.

Les substances bactéricides inhibent le développement de la flore cutanée responsable des odeurs axillaires. Parmi ces produits bactéricides, on peut citer le triclosan (5-chloro-2-(2,4-dichlorophénoxy) phénol).

Les absorbeurs d'odeurs sont des produits qui soient capables de capturer et retenir en leur sein les molécules responsables des mauvaises odeurs (cette définition est donnée dans l'ouvrage « Cosmetic Science and Technology Series » - 1988 /Volume 7 chap.10 - IIIc). Ces produits ont pour effet de diminuer plus ou moins fortement l'odeur de sueur déjà développée. Parmi les absorbeurs d'odeurs, on peut citer les sels de zinc, notamment les sels de zinc de polyacides carboxyliques décrits dans le brevet US 4 425 321 et en particulier ceux de l'acide dimerginique et ceux de l'acide trimerginique hydroxylé ou non hydroxylé. On peut également citer les sels de zinc hydrosolubles.

Pour une utilisation comme composition capillaire, la composition selon l'invention, du fait des propriétés rhéologiques spécifiques de la matrice solide la constituant, apporte un bon effet coiffant et de la discipline dans la coiffure.

Pour obtenir un effet fixant ou améliorer l'effet coiffant et démêlant, on peut ajouter à la composition selon l'invention un matériau fixant ou un matériau conditionneur. Ces matériaux fixants ou conditionneurs peuvent être employés dans des quantités comprises entre 0,01 et 15 % en poids par rapport au poids total de la composition, de préférence entre 0,1 et 8 % en poids.

La composition selon l'invention peut également comprendre des actifs pour le soin des cheveux et/ou des agents de renfort de brillance et/ou des agents de coloration capillaire. Ces actifs et/ou agents capillaires peuvent être employés dans des quantités comprises entre 0,01 et 20 % en poids par rapport au poids total de la composition selon l'invention.

Les matériaux fixants utiles selon l'invention sont essentiellement constitués par au moins un polymère fixant, seul ou en combinaison avec des additifs cosmétiques usuels, par exemple des plastifiants, ou des agents neutralisants. Selon l'invention, on peut utiliser tout polymère fixant connu en soi. On peut utiliser en particulier un polymère fixant choisi parmi les polymères anioniques, cationiques, amphotères, non ioniques et leurs mélanges. Les polymères fixants anioniques ou amphotères peuvent être si nécessaire neutralisés partiellement ou totalement. Les agents de neutralisation sont par exemple la soude, la potasse, I'amino-2 méthyl-2 propanol-1, la monoéthanolamine, la triéthanolamine ou la triisopropanolamine, les acides minéraux ou organiques tels que l'acide chlorhydrique ou l'acide citrique.

Les polymères fixants peuvent être utilisés sous forme solubilisée ou sous forme de dispersions de particules solides de polymère.

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 et 3.000.000.

Les polymères fixants anioniques généralement utilisés sont des polymères comportant des groupements dérivés d'acide carboxylique, sulfonique ou phosphorique et ont un poids moléculaire moyen en poids compris entre environ 500 et 5.000.000. De tels polymères sont notamment décrits dans les brevets et demandes de brevet DE 2.330.956, FR 1.222.944, FR 1.564.110, FR 1.580.545, FR 2.198.719, FR 2.265.782, FR 2.265.781, FR 2.350.384, FR 2.357.241, FR 2.439.798, GB 839.805, LU 75370, LU 75371, US 2.047.398, US 2.723.248, US 2.102.113 et US 4.128.631. Ils sont notamment choisis parmi les produits commercialisés sous les dénominations VERSICOL® E ou K par la société ALLIED COLLOID, AMERHOLD® DR 25 par la société AMERCHOL, QUADRAMER® par la Société AMERICAN CYANAMID, ARISTOFLEX® A, LUVIFLEX® VBM 70, LUVIMER® 100 P ou MAEX ou MAE, ULTRAHOLD® et ULTRAHOLD® STRONG par la société BASF, COSMEDIA® POLYMER HSP 1180 par la société HENKEL, RETEN® 421, 423 ou 425 par la Société HERCULES; ACRYLIDONE® LM, GANTREZ® AN ou ES et AVANTAGE® CP par la société ISP, Flexan® 500, Flexan® 130 et RESINES 28-29-30, 26-13-14 ou 28-13-10 par la société NATIONAL STARCH, ACUDYNE® 255 par la société ROHM & HAAS, EUDRAGIT® L par la société ROHM PHARMA et STEPANHOLD® EXTRA par la société STEPAN, ou le copolymère acide crotonique / acétate de vinyle / t.butylbenzoate de vinyle de la société CHIMEX.

Les polymères fixants amphotères utilisables conformément à l'invention sont notamment décrits dans les brevets FR 1 400 366 et US 3 836 537. Ils sont notamment choisis parmi les produits désignés par la dénomination CTFA (4ème Ed., 1991) Octylacrylamide / acrylates / butylaminoethylmethacrylate copolymer et ceux commercialisés sous les dénominations AMPHOMER® AMHOMER® LV 71 ou LOVOCRYL® 47 par la société NATIONAL STARCH, DIAFORMER® Z301 par la société SANDOZ et EVALSAN® par la société JAN DEKKER

Les matériaux conditionneurs utiles selon l'invention sont essentiellement constitués par les matériaux conditionneurs usuels en cosmétique. Il sont notamment choisis parmi les agents tensio-actifs cationiques, les polymères anioniques ou non ioniques ou cationiques ou amphotères, les protéines, les hydrolysats de protéines, les céramides, les pseudocéramides, les acides gras à chaînes linéaires ou ramifiées en C₁₆-C₄₀ tels que l'acide méthyl-18 eicosanoique, les silicones volatiles ou non volatiles, solubles et insolubles dans le milieu et leurs mélanges.

Les agents conditionneurs de type polymères cationiques utiles selon l'invention peuvent être choisis parmi tous ceux déjà connus en soi comme améliorant les propriétés cosmétiques des cheveux, à savoir notamment ceux décrits dans les demandes de brevet EP 0 337 354, FR 2 270 846, FR 2 383 660, FR 2 598 611, FR 2 470 596 et FR 2 519 863. Parmi tous les polymères cationiques susceptibles d'être utilisés dans le cadre de la présente invention, on préfère mettre en oeuvre les dérivés d'éther de cellulose quaternaires tels que les produits commercialisés sous la dénomination JR 400® par la société UNION CARBIDE CORPORATION, les cyclopolymères, en particulier les homopolymères de sel de diallyldiméthylammonium et les copolymères de sel de diallyldiméthylammonium et d'acrylamide en particulier les chlorures, commercialisés sous les dénominations MERQUAT® 100, MERQUAT® 550 et MERQUAT® S par la société MERCK, les polysaccharides cationiques et plus particulièrement les gommes de guar modifiées par du chlorure de 2,3-époxypropyl triméthylammonium commercialisées par exemple sous la dénomination JAGUAR C13S® par la société MEYHALL.

Parmi les agents de renfort de brillance, on peut citer les silicones arylées non volatiles, en particulier les polyalkylarylsiloxanes telles que Silicone phénylée commercialisée sous la dénomination DC 556 par la société DOW CORNING - Diphényldiméthicone commercialisée sous la dénomination MIRASIL® DPDM par la société RHONE POULENC.

Parmi les agents de coloration capillaire, on peut citer en particulier les agents de coloration directe. Parmi ceux classiquement utilisés, on peut citer des colorants nitrés benzéniques, tels que les nitrophénylènediamines, les nitrodiphénylamines, les nitroanilines, les éthers de phénol nitrés ou les nitrophénols, des nitropyridines, des colorants anthraquinoniques, mono- ou di-azoïques, triarylméthaniques, aziniques, acridiniques et xanthéniques ou encore des colorants métallifères. Ces agents de coloration directe, sous forme de base ou salifiée, sont généralement présents dans la composition selon l'invention dans des proportions pouvant aller d'environ 0,001 à environ 10%, et de préférence d'environ 0,05 à environ 5% en poids par rapport au poids total de la composition.

Pour une utilisation comme composition de maquillage, la composition selon l'invention peut comprendre des matériaux usuels pour les compositions de maquillage, en particulier des charges et/ou des matériaux colorants.

De manière préférentielle, la quantité de charges présentes dans la composition selon l'invention est comprise entre 0,01 et 60 % en poids par rapport au poids total de la composition, plus préférentiellement comprise entre 2 et 30 % en poids.

La quantité de matériaux colorants est de préférence comprise entre 0,01 et 30 % en poids par rapport au poids total de la composition, plus préférentiellement comprise entre 1 et 15 % en poids.

Les charges sont des matériaux naturels ou synthétiques dont la fonction principale consiste à modifier les propriétés physico-chimiques (rhéologiques, mécaniques, optiques) et/ou cosmétiques d'une composition. Parmi les charges, on peut citer le talc, le mica, la silice, le kaolin, les poudres de Nylon® (Orgasol notamment) et de polyéthylène, le Téflon®, l'amidon, le nitrure de bore, des microsphères de copolymères, telles que l'Expancel® (Nobel Industrie), le polytrap® (Dow Corning) et les microbilles de résine de silicone (Topsearls® de Toshiba, par exemple).

Les matériaux colorants sont des matériaux naturels ou synthétiques utilisés pour donner à une matière une coloration durable. On distingue d'une part les colorants et d'autres part les pigments. Les colorants sont des substances (ou matériaux) naturelles ou de synthèse essentiellement solubles dans leur milieu d'utilisation, dont la fonction principale consiste à donner une coloration. On peut citer les colorants organiques naturels, tels que le carmin de cochenille (CI 75 470), ou de synthèse, tels que les colorants halogéno-acides, azoïques, anthraquinoniques. On peut également citer des colorants minéraux tels que le sulfate de cuivre. Les pigments sont des substances (ou matériaux) naturelles ou de synthèse constituées de fines particules qui, par opposition aux colorants, sont insolubles dans leur milieu d'utilisation, dont la fonction principale consiste à donner une coloration. On distingue différents types de pigments, les pigments minéraux, les pigments organiques, les laques ou les pigments nacrés.

Les laques sont des colorants adsorbés sur des particules insolubles, l'ensemble restant insoluble dans leur milieu d'utilisation. Les pigments nacrés sont des substances (ou matériaux) naturels ou synthétiques, qui diffractent et réfléchissent la lumière pour donner un effet irisé ou brillant.

Parmi les pigments minéraux, on peut citer les oxydes métalliques, en particulier les oxydes de zirconium, de cérium, de zinc ou de chrome (CI 77 288), le dioxyde de titane (CI 77 891), les oxydes de fer noir, jaune, rouge et brun (CI 77 499, CI 77492, CI 77491), le violet de manganèse (CI 77 742), le bleu outremer (CI 77 007), le bleu ferrique (CI 77 510) , l'hydrate de chrome (CI 77 289), la poudre d'argent ou la poudre d'aluminium.

Parmi les pigments organiques, on peut citer le noir de carbone (CI 77 266), ou le D & C Rouge 36. Les laques sont généralement constituées par des sels métalliques (AI, Zr, Ca, Na) de colorants organiques, adsorbés sur des particules d'alumine. On peut citer celles connues sous les dénominations: D & C Red 21 (CI 45 380), D & C Orange 5 (CI 45 370), D & C Red 27 (CI 45 410), D & C Orange 10 (Cl 45 425), D & C Red 3 (CI 45 430), D & C Red 7 (CI 15 850:1), D & C Red 4 (CI 15 510), D & C Red 33 (CI 17 200), D & C Yellow 5 (CI 19 140), D & C Yellow 6 (CI 15 985), D & C Green 5 (CI 61 570), D & C Yellow 10 (CI 77 002), D & C Green 3 (CI 42 053), D & C Blue 1 (CI 42 090).

Parmi les pigments nacrés, on peut citer le mica recouvert d'oxyde de titane, d'oxychlorure de bismuth, d'oxyde de fer, ou de pigments naturels, par exemple le mica titane coloré.

Lorsque la composition est une composition de soin, elle comprend en outre des actifs comme l'acide ascorbique, l'acide kojique, l'acide citrique, l'acide caféique, l'acide salicylique et ses dérivés (par exemple acide n-octanoyl-5 ou décanoyl-5-salicylique), les α-hydroxyacides, l'acide rétinoïque et ses dérivés tels que le rétinol et les esters de rétinol, l'acide benzène 1,4-di(3-méthylidène 10-camphosulfonique). On peut aussi, utiliser tous les composés naturels ou synthétiques contenant de tels acides, comme les extraits végétaux et plus spécialement les extraits de fruits, ou encore les dérivés xanthiques (caféine, théophylline), l'acide β-glycyrrhétinique ou l'acide asiatique. On peur également employer des actifs hydratants comme des polyols, en particulier de la glycérine, ou encore des actifs tenseurs comme des protéines d'origine végétale.

En fonction de l'utilisation envisagée et des propriétés physico-chimiques et/ou cosmétiques de la composition selon l'invention, elle peut également comprendre des gélifiants additionnels de manière à modifier ses propriétés de douceur ou de dureté, elle peut comprendre également des latex ou pseudolatex qui augmentent la rémanence à l'eau de la composition et lui apportent plus de brillance, ou encore une ou plusieurs gommes de silicone qui permettent de conférer aux compositions finales des qualités de douceur et de glissant.

Parmi les gélifiants pouvant être employés dans la composition selon l'invention, on peut citer les extraits d'algue tels que l'agar-agar, les carraghénanes, les alginates; les extraits de graines tels que la gomme de caroube, la gomme de guar; les exudats de plantes tels que la gomme arabique, la gomme karaya, la gomme adragante, la gomme de gatty; les exudats de microorganismes tels que la gomme de xanthane, la cellulose ou ses dérivés comme la carboxyméthylcellulose, l'hydroxypropylcellulose, la méthylcellulose, l'hydroxypropylméthylcellulose ou l'hydroxyéthylcellulose ainsi que les celluloses modifiées notamment par greffage de groupement alkyle; les extraits de fruits tels que les pectines; les agents gélifiants d'origine animale tels que la gélatine, les caséïnates; les polymères synthétiques gélifiants hydrosolubles tels que les acides polyacryliques réticulés tels que les « Carbopol » ou « Pemulen » commercialisés par la société GOODRICH; les dérivés du silicium tels que les hectorites synthétiques comme les produits « Laponite RD et RDS » commercialisés par la société WAVERLY, les silicates d'aluminium et de magnésium comme le produit « Veegum» commercialisé par la société VANDERBILT. Il est entendu que ces gélifiants additionels ajoutés à la composition solide selon l'invention ne viendront pas modifier les propriétés rhéologiques de la matrice solide la constituant.

L'homme du métier saura déterminer le procédé de préparation de la composition selon l'invention en fonction de ses constituants. Dans la mesure où la matrice solide la constituant présente une viscosité qui diminue fortement sous une contrainte supérieure ou égale à la contrainte de cisaillement C₁, il est possible de mélanger à une température appropriée les différents éléments de la composition, puis une fois l'homogénéité recherchée obtenue, donner une forme à la composition selon une méthode usuelle, notamment en la laissant reposer dans un moule correspondant à ladite forme.

La présente invention concerne également l'utilisation cosmétique d'une composition telle que définie ci-dessus.

Elle concerne enfin un procédé de traitement cosmétique de la peau, des muqueuses, des cheveux ou des phanères dans lequel on applique la composition telle que définie ci-dessus sur la peau, les muqueuses, les cheveux ou les phanères.

Les exemples ci-après permettent d'illustrer la présente invention sans toutefois chercher à en limiter la portée. Les pourcentages sont exprimés en poids par rapport au poids total des composition. Les pourcentages de copolymère isophtalate / téréphtalate / sulfoisophtalate d'éthylène glycol dans les compositions sont des pourcentages de matière active.

### Exemple 1 Préparation d'un oligomère copolyester téréphtalique

Dans un réacteur en acier inoxydable de 7,5 litres, muni d'un agitateur à ancre tournant à 80 tr/mn relié à un couplemètre KYOWA, d'une double enveloppe pour la circulation d'un liquide caloporteur et d'une colonne à distiller régulée par une électrovanne on introduit:
- 11,47 moles de téréphtalate de diméthyle
- 2,53 moles de diméthyl-5 sulfonate de sodium
- 39,16 moles d'éthylène glycol
- 54 ppm en poids de titane, sous forme d'aminotriéthanolate de titane comme catalyseur et agent limiteur d'éthérification.

Le mélange est préchauffé à 180°C. Il est ensuite porté jusqu'à la température de 220°C en environ 130 minutes, pour distiller plus de 90% de la quantité théorique de méthanol.

Le mélange réactionnel est ensuite amené à 230°C en 30 minutes. Lorsque la masse réactionnelle a atteint cette température, on introduit en 60 minutes, toujours à 230°C, une suspension dont la composition est la suivante :
- 0,5 mole d'acide isophtalique
- 2,36 moles d'acide téréphtalique
- 8 moles d'éthylène glycol

La masse réactionnelle est alors amenée à la température de 250°C en 60 minutes.

Pendant la période d'introduction du mélange et pendant la période de chauffage jusqu'à 250°C, on distille un mélange d'eau et d'éthylène glycol sans rétrogradation.

Le mélange réactionnel est ensuite transféré dans un autoclave préchauffé à 250°C puis mis sous pression réduite de 100 millibar en 22 minutes. Après 2 minutes dans ces conditions de température et de pression, la masse réactionnelle est coulée et refroidie.

Le copolyester obtenu présente les caractéristiques structurales décrites au tableau 1 ci-après, dans lequel:
* "% molaire des motifs diacides" correspond à la teneur, en %, de chaque diacide ou diester mise en oeuvre par rapport à l'ensemble des diacides ou diesters mis en oeuvre.

"Tp" signifie : motif téréphtalique
"Ip" signifie : motif isophtalique
"Slp" signifie : motif sulfoisophtalique

* Les caractéristiques de la partie "glycol" des copolyesters sont obtenues par méthanolyse des produits à 190°C pendant 16 heures suivies d'une analyse par la technique de chromatographie en phase vapeur et dosage par étalonnage interne.
- "% molaire des motifs diols" correspond à la teneur, en %, des motifs oxyéthylène "G", des motifs di(oxyéthylène) "2G", des motifs tri(oxyéthylène) "3G" et des motifs tetra(oxyéthylène) "4G", par rapport à l'ensemble des motifs diols.
- "%GT/Σ motifs" correspond au % molaire des motifs de formule (I)

   [-CO-A-CO-O-(CH₂-CH₂-O)ₙ-] (I)

   où A est 1,4 phénylène et n=1 par rapport à l'ensemble des motifs de formule (I) où A est 1,4 phénylène, sulfo1,3 phénylène et éventuellement 1,3 phénylène et n va de 1 à 4 "%GT/Σ motifs" se calcule par la formule suivante :

   %GT/Σ motifs = (% molaire de motifs Tp) x (% molaire de motifs G) / 100

* La masse molaire des polyesters (Mw) est déterminée par chromatographie par permation de gel (GPC) dans le DMAc/LiBr à 100%, les résultats sont données en équivalents polystyrène.

| % molaire des motifs diacides | |
|---|---|
| Tp | 82 |
| Ip | 3 |
| Slp | 15 |
| %GT/Σ motifs | 46,5 |

| % molaire des motifs diols | |
|---|---|
| G | 56,8 |
| 2G | 30,7 |
| 3G | 10 |
| 4G | 2,5 |
| Mw | 8 000 |

### Exemple 2 Stick de coiffage

On prépare un gel aqueux en mélangeant à froid 20% d'oligomère de l'exemple 1 et le complément à 100 % d'eau déminéralisée. Le gel fluide obtenu est coulé dans un moule et laissé reposer 24 heures. Après prise en masse, on obtient un stick de coiffage présentant les propriétés rhéologiques suivantes:
Viscosité initiale V₀: 90000 Pa.s
Contrainte de cisaillement C₁ : 500 Pa
Viscosité V₂ a une contrainte de 1500 Pa: 30 Pa.s.

### Exemple 3 Stick de coiffage

On reprend le mode opératoire de l'exemple 2 avec 25 % d'oligomère de l'exemple 1 et 3 % de copolymère polyvinyle pyrrolidone / acétate de vinyle. On obtient un stick de coiffage présentant des propriétés rhéologiques comparables à celles du produit de l'exemple 2.

### Exemple 4 Stick de brillance

On reprend le mode opératoire de l'exemple 2 avec 25 % de l'exemple 1 et 10 % d'huile 70641 V200 de la société RHONE POULENC. On obtient un stick de brillance présentant des propriétés rhéologiques comparables à celles du produit de l'exemple 2.

### Exemple 5 Stick déodorant

On reprend le mode opératoire de l'exemple 2 avec 20 % d'oligomère de l'exemple 1 et 0,25 % de triclosan. On obtient un stick déodorant présentant des propriétés rhéologiques comparables à celles du produit de l'exemple 2.

### Exemple 6 Rouge à lèvres

On reprend le mode opératoire de l'exemple 2 avec 20 % d'oligomère de l'exemple 1, 5 % de pigments et 1 % de conservateur. On obtient un stick de rouge à lèvres aqueux présentant des propriétés rhéologiques comparables à celles du produit de l'exemple 2. Le stick se dépose facilement sur les lèvres, procure une sensation de fraicheur à l'application, et forme un film sans transfert et brillant.

## Revendications

1. Composition topique solide aqueuse constituée par une matrice solide aqueuse ayant l'aspect d'un gel, caractérisée en ce que la matrice présente le profil rhéologique suivant:
- une viscosité initiale au repos V₀ suffisante pour former une composition solide, la dite viscosité V₀ étant stable jusqu'à une contrainte de cisaillement C₁
- une viscosité V₂ après cisaillement à une contrainte C₂ pour laquelle le rapport V₀/V₂ est supérieur ou égal à 1000,
- la différence C₂ -C₁ étant inférieure ou égale à 1000 Pa.

2. Composition selon la revendication 1, caractérisée en ce que la viscosité initiale V₀ est comprise entre 50000 et 1000000 Pa.s.

3. Composition selon l'une des revendications 1 ou 2, caractérisée en ce que le gel présente à la contrainte de début de cisaillement C₁ une viscosité V₁, le rapport V₀/V₁ étant inférieur ou égal à 2.

4. Composition selon l'une des revendications 1 à 3, caractérisée en ce que la contrainte de cisaillement C₁ est supérieure ou égale à 500 Pa, et

5. Composition selon l'une des revendications 1 à 4, caractérisée en ce que les chutes de viscosité induites par le cisaillement sur la composition ne sont pas immédiatement réversibles.

6. Composition selon l'une des revendications 1 à 5, caractérisée en ce que la matrice comprend un matériau gélifiant hydrophile.

7. Composition selon la revendication 6, caractérisée en ce que le matériau gélifiant hydrophile est un polymère gélifiant hydrophile.

8. Composition selon la revendication 7, caractérisée en ce que le polymère gélifiant hydrophile est un oligomère copolyester terephtalique hydrosoluble ou hydrodispersable comprenant essentiellement des motifs répétitifs dicarboxylates de formule (I):
[-CO-A-CO-O-(CH₂-CH₂)ₙ-] (I)
dans laquelle
A représente un groupement 1,4-phénylène, sulfo-1,3-phénylène et éventuellement 1,3-phénylène,
n va de 1 à 4,
au moins 35 % en mole, préférentiellement au moins 40 % en mole, plus préférentiellement entre 40 et 90 % en mole desdits motifs de formule (I) étant des motifs de formule (I) pour lesquels A représente un groupement 1,4-phénylène et n est égal à 1,
au moins 7 % en mole, préférentiellement au moins 10 % en mole, plus préférentiellement entre 10 % et 25 % en mole desdits motifs de formule (I) étant des motifs de formule (I) pour lesquels A représente un groupement sulfo-1,3-phénylène,
l'oligomère copolyester pouvant comprendre éventuellement jusqu'à 20 % en mole, de préférence jusqu'à 5 % en mole de motifs de formule (I) pour lesquels A représente un groupement 1,3-phénylène, et
la masse moléculaire en poids dudit oligomère copolyester étant inférieure à 20 000, de préférence inférieure à 15 000.

9. Composition selon la revendication 8, caractérisée en ce que l'oligomère copolyester a une masse moléculaire en poids comprise entre 5 000 et 14 000, plus préférentiellement comprise entre 8 000 et 10 000.

10. Composition selon l'une des revendications 1 à 9, caractérisée en ce qu'elle comprend entre 10 et 40 % en poids par rapport au poids total de la composition de matériau gélifiant hydrophile, de préférence entre 10 et 30 % en poids.

11. Composition selon l'une des revendications 1 à 10, caractérisée en ce qu'elle est destinée à être appliquée sur la peau, les muqueuses ou les phanères.

12. Composition selon l'une des revendications 1 à 11, caractérisée en ce qu'elle contient des additifs et/ou des actifs usuels en dermo-cosmétique choisis dans le groupe constitué par des parfums ; des stabilisants comme des conservateurs; des filtres U.V.A et/ou U.V.B. ; des antioxydants hydrophiles et/ou lipophiles ; des chélatants; des actifs hydrophiles et/ou lipophiles, en particulier des agents anti-radicaux libres, des alpha- ou bêta-hydroxyacides, des céramides; des agents antipelliculaires, des agents antiacnéiques, des agents anti-chute des cheveux, des agents antifongiques ou antiseptiques, des substances actives de type antitranspirant et/ou de type bactéricide et/ou absorbeurs d'odeurs; des matériaux fixants ou des matériaux conditionneurs des cheveux; des actifs pour le soin des cheveux et/ou des agents de renfort de brillance et/ou des agents de coloration capillaire ; des charges ; des matériaux colorants ; des gélifiants ; des gommes de silicone.

13. Composition selon l'une des revendications 1 à 12, caractérisée en ce qu'il s'agit d'une composition d'hygiène corporelle.

14. Composition selon l'une des revendications 1 à 12, caractérisée en ce qu'il s'agit d'une composition capillaire.

15. Composition selon l'une des revendications 1 à 11, caractérisée en ce qu'il s'agit d'une composition de maquillage.

16. Composition selon la revendication 15, caractérisée en ce qu'il s'agit d'une composition de maquillage dit « sans transfert ».

17. Composition selon l'une des revendications 15 ou 16, caractérisée en ce qu'il s'agit d'un rouge à lèvres.

18. Composition selon l'une des revendications 1 à 11, caractérisée en ce qu'il s'agit d'une composition de soin.

19. Procédé de traitement cosmétique de la peau, des muqueuses, des cheveux ou des phanères dans lequel on applique une composition selon l'une des revendications 1 à 18 sur la peau, les muqueuses ou les phanères.
